# EUROPÄISCHE PATENTSCHRIFT

(11) **EP 1 223 927 B1**
(45) Veröffentlichungstag und Bekanntmachung des Hinweises auf die Patenterteilung: **09.02.2005**
(21) Anmeldenummer: 00969283.1
(22) Anmeldetag: 22.09.2000
(51) Int. Cl.: A61K 31/325, A61P 29/02

(54) **VERWENDUNG VON RETIGABIN ZUR BEHANDLUNG VON NEUROPATHISCHEN SCHMERZEN**
USE OF RETIGABIN FOR TREATING NEUROPATHIC PAIN
UTILISATION DE RETIGABINE POUR LE TRAITEMENT DE DOULEURS NEUROPATHIQUES

(30) Priorität: 27.09.1999 US 406135
(43) Veröffentlichungstag der Anmeldung: 24.07.2002
(73) Patentinhaber: Xcel Pharmaceuticals, Inc., San Diego, CA 92122 (US)
(72) Erfinder: RUNDFELDT, Chris, 01640 Coswig (DE); BARTSCH, Reni, 01458 Ottendorf-Okrilla (DE); ROSTOCK, Angelika, 01445 Radebeul (DE); TOBER, Christine, 01689 Weinböhla (DE); DOST, Rita, 01217 Dresden (DE)
(74) Vertreter: Vossius & Partner
(86) Internationale Anmeldenummer: PCT/EP2000/009284
(87) Internationale Veröffentlichungsnummer: WO 2001/022953

(56) Entgegenhaltungen:
- WO-A-01/01970
- DE-A- 4 200 259
- ROSTOCK A ET AL: "Effects of the anticonvulsant retigabine in neuropathic pain models in rats." ARCH.PHARMACOL. (361, NO. 4, SUPPL., R99, 8 MARCH 2000) CODEN: NSAPCC ISSN: 0028-1298, XP001028668 Asta-Medica

## Beschreibung

Die Erfindung betrifft die Verwendung von 2-Amino-4-(4-fluorbenzylamino)-1-ethoxycarbonylaminobenzen der Formel I 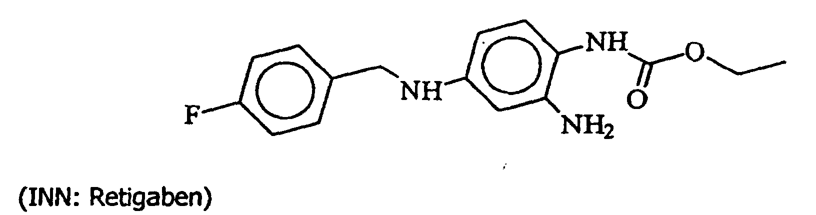 oder deren pharmazeutisch verwendbaren Salze zur Prophylaxe und Behandlung von bestimmten neuropathischen Schmerzen.

In der WO 01/01970 wird die Verwendung von Retigabin zur Behandlung von neuropathischen Schmerzen und Migräne angesprochen. Bei diesem Dokument handelt es sich um Stand der Technik gemäß Artikel 54(3) und (4) EPÜ.

Neuropathischer Schmerz wie Allodynie und Hyperalgesie bezeichnet eine besondere Art der Schmerzwahmehmung, die sich von der üblichen Wahrnehmung von schmerzhaften Reizen unterscheidet. Patienten, die unter hyperalgetischem Schmerz leiden, empfinden schmerzhafte Reize stärker als gesunde Personen. Der Begriff Allodynie bezeichnet das Phänomen der Wahrnehmung von an sich nicht schmerzhaften Reizen wie Berührung oder Wärme/Kälte als Schmerz. Die empfundenen Wahrnehmungen sind teilweise sehr stark und belastend.

Diese veränderte Schmerzwahrnehmung wird im deutschen und internationalen Sprachgebrauch mit verschiedenen Begriffen belegt, die sich teilweise in ihrer Bedeutung überlappen aber nicht immer synonym verwendet werden können. Im deutschen Sprachgebrauch sind die Begriffe Allodynie, Parästhesie, Hyperestesie, Hyperalgesie und Phantomschmerz üblich, im englischen Sprachgebrauch werden neben "allodynia, hyeralgesia and pantom limb pain" weiterhin die Begriffe "reflex sympathetic dystrophy" (RSD) (Rogers and Valley, 1994) und "sympathetically maintained pain" (SMP) verwendet (Rogers-JN; Valley-MA, Reflex sympathetic dystrophy; Clin-Podiatr-Med-Surg. 1994 Jan; . 11(1): 73-83).

Als Allodynie wird die verstärkt unangenehm bis schmerzhafte Wahrnehmung von thermischen oder durch Berührung ausgelösten Reizen verstanden, die auf einer Senkung der Schmerzschwelle nur für diese Stimuli beruht. Hyperalgesie bezeichnet die übermäßige Wahrnehmung von an sich schmerzhaften Reizen verschiedenster Art, wieder aufgrund einer Senkung der Schmerzschwelle. Als Phantomschmerz wird die Wahrnehmung von Schmerzen bezeichnet, die nicht existent sind, da beispielsweise die schmerzhafte Extremität amputiert wurde. In der wissenschaftlichen Literatur wird diese Art der Schmerzwahrnehmung oft unter dem Begriff des zentral vermittelten neuropathischen Schmerzes subsummiert. Charakteristisch ist dabei, daß die eigentliche Schmerzwahmehmung nicht auf einen üblichen Schmerz auslösenden Reiz zurückzuführen ist, sondern vom peripheren oder zentralen Nervensystem generiert wird, da die Reaktionslage des schmerzempfindenden und schmerzweiterleitenden Systems verändert ist. Im Gegensatz zu anderen Schmerzformen ist neuropathischer Schmerz meist chronisch und ist üblicherweise mit klassischen Analgetika wie Opioiden nicht oder nur schlecht zu behandeln.

Erkrankungen, bei denen eine veränderte Reaktionslage des schmerzempfindenden und schmerzweiterleitenden Systems beobachtet wird, können sein:
1. Eine klassische Folge der Herpes zoster (Gürtelrose)-Infektion ist eine lang anhaltende Allodynie beschrieben (Fields et al., 1998; Fields-HL; Rowbotham-M; Baron-R, Postherpetic neuralgia: irritable nociceptors and deafferentation. Neurobiol-Dis. 1998 Oct; 5(4): 209-27).
2. Bei AIDS-Patienten treten in verschiedenen Stadien der Erkrankung Schmerzempfindungen auf, die zu dem Formenkreis der Hyperalgesie gehören und klar von nozizeptivem (d.h. durch schmerzhafte Reize ausgelöstem) Schmerz zu unterscheiden sind, Lefkowitz 1996; Lefkowitz-M, Pain management for the AIDS patient. J-Fla-Med-Assoc. 1996 Dec; 83(10):701-4).
3. Verbrennungswunden führen in den betroffenen Körperteilen zu neuropathischen Hyperalgesien. Obwohl die Schmerz auslösende Ursache (Hitze) nicht mehr vorhanden ist, sind Verbrennungswunden oft äußerst schmerzhaft.
4. Nach Therapie mit hohen Dosen von Zytostatika zur Krebsbehandlung berichten Patienten oft auch von Schmerzempfindungen (Brant 1998; Brant-JM, Cancer-related neuropathic pain. Nurse-Pract-Forum. 1998 Sep; 9(3): 154-62). Tanner et al. (Tanner-KD; Reichling-DB; Levine-JD, Nociceptor hyper-responsiveness during vincristine-induced painful peripheral neuropathy in the rat J-Neurosci. 1998 Aug 15; 18(16): 6480-91) konnten zeigen, daß Schmerzen, die im Zusammenhang mit der Vncristin-Behandlung auftreten, durch eine erhöhte Erregbarkeit der peripheren Schmerzrezeptoren, das heißt durch eine Hyperalgesie verursacht ist.
5. Eine Tumorerkrankung selbst kann auch (z.B. durch chronische Nervenkompression durch den Tumor neuropathische Schmerzen auslösen, die in den Formenkreis der Hyperalgesie gehören (Brant 1998; Brant-JM, Cancer-related neuropathic pain. Nurse-Pract-Forum. 1998 Sep; 9(3): 154-62).
6. Bei Diabetespatienten kommt es häufig im Verlauf der Erkrankung als eine Form der Spätschädigung zu Hyperalgesie. Die Patienten klagen über stark schmerzende Gliedmaßen bei gleichzeitig reduzierter Berührungsempfindlichkeit der Haut (Bell 1991; Bell-DDS: Lower limb problems in diabetic patients. What are the causes? What are the remedies? Postgrad-Med. 1991 Jun; 89(8): 237-40, 243-4).
7. Ein weiteres Erkrankungsbild, in dem Symptome der Hyperalgesie und Allodynie auftreten, ist die Vulvodynie. Diese Erkrankung ist durch chronisches Missempfinden (Brennen, Stechen, Jucken) im Bereich der Vulva charakterisiert, ohne dass Infektionserreger als Ursache nachgewiesen werden können (Bohl et al., 1998; Bohl-TG, Vulvodynia and ist differential diagnoses. Semin-Cutan-Med-Surg. 1997 Sep; 17(3): 189-95).
8. Bei Patienten mit chronischen Rückenschmerzen kann oft eine Kompression von Nervenwurzeln des Rückenmarks beobachtet werden. Diese Druckschädigung der Nervenwurzeln äußert sich außer in chronischen Schmerzen auch in sensorischen Missempfindungen (Parästhesien). Wird chirurgisch die Einengung beseitigt, klagt trotzdem ein großer Anteil der Patienten weiterhin über Schmerzempfindungen. Diese peristierenden Empfindungen werden als neuropathische Schmerzen bezeichnet und lassen sich diagnostisch von anderen (entzündlichen) Schmerzformen abgrenzen (Sorensen and Bengtsson, 1997; Sorensen-J; Bengtsson-M, Intravenous phentolamine test―an aid in the evaluation of patients with persistent pain after low-back surgery? Acta-Anaesthesiol-Scand. 1997 May; 41(5): 581-5).
9. Bei 10 bis 20 % Patienten mit Rückenmarksverletzungen entstehen teilweise sehr starke Schmerzempfindungen, die mangels intaktem Rückenmark im Gehirn generiert werden und nicht einem schmerzhaften Stimulus zuzuordnen sind. Dieser Schmerz wird als zentraler neuropathischer Schmerz bezeichnet (Eide 1998; Eide-PK Pathophysiological mechanisms of central neuropathic pain after spinal cord injury. Spinal-Cord, 1998 Sep; 36(9): 601-12).
10. Nach Amputation auftretender Schmerz weist Charakteristika von neuropathischem Schmerz auf (Hill 1999; Hill-A. Phantom limb pain: a review of the literature on attributes and potential mechanisms. J-Pain-Symptom-Manage. 1999 Feb; 17(2): 125-42).
11. Auch innere Organe können Quelle einer Hyperalgesie sein (Mayer and Gephart, 1994; Mayer-EA; Gebhart-GF, Basic and clinical aspects of visceral hyperalgesie [see comments in: Gastroenterology 1995 Feb; 180(2): 618] Gastroenterology. 1994 Jul; 107(1): 271-93). Betroffene Patienten leiden unter nicht angemessenen Wahrnehmungen von physiologischen Reaktionen in verschiedenen Bereichen des Magen-Darmtraktes wie z. B. Völlegefühl, Magenschmerzen oder das Gefühl von Blähungen, ohne dass entsprechende pathologische Ursachen vorliegen.

Wie eingangs erwähnt, ist eine erhöhte oder veränderte Schmerzreaktion ein Symptom vielfältiger Erkrankungen und es erscheint fraglich, ob eine einheitliche Pathogenese vorliegt. Dies zeigt sich auch darin, daß die Art der veränderten Schmerzreaktion sehr unterschiedlich sein kann. Gemeinsam ist allen diesen Schmerzreaktionen jedoch, daß Morphine entweder unwirksam sind oder erst bei Verwendung von Dosierungen, die unerwünschte Nebenwirkungen auslösen, wirken. Auslösende Faktoren für die Schmerzreaktion können vielfältig sein.

Bei Patienten mit Herpes-induzierter Allodynie kann ein Luftzug ausreichen um brennende Schmerzen auszulösen. Bei diesen Patienten ist davon auszugehen, daß die Erreger in Neuronen eine Schädigung hervorrufen, die die Schmerzschwelle senkt. Bei Patienten mit Diabetes vermutet man, daß die Minderversorgung der Nerven mit Blut und Nährstoffen aufgrund der Mikroangiopathie zu einer chronischen Nervenschädigung führt. Diese löst wiederum einen Regenerationsprozess aus, der sich in Sprossungen von Nervenfasern äußert.

Reorganisationsprozesse im Rückenmark wie auch peripher werden von verschiedenen Autoren als mögliche Ursache von Hyperalgesien angesehen (siehe z.B. Basbaum 1999; Basbaum-Al Spinal mechanisms of acute and persistent pain. Reg-Anesth-Pain-Med. 1999 Jan-Feb; 24(1): 59-67). Durch chronische Kompression von Nerven werden diese geschädigt ohne vollständig zerstört zu werden. Während durch akute Kompression lokal Schmerz-Signale ausgelöst werden, kommt es bei chronischer Kompression zu einer Induktion von Transskriptionsfaktoren im Zellkörper (und damit außerhalb des Bereichs der Kompression im Rückenmark), die über Wochen anhält. Die Neuropeptide wie Substanz P aktivieren die Aussprossung von Nervenfasern und die Aktivierung nicht betroffener benachbarter Neurone. Zudem konnte nachgewiesen werden, daß die Nervenzellkörper vermehrt Noradrenalinrezeptoren exprimieren. Dadurch können die Neurone spontan ohne äußeren Anstoß aktiv werden und spontan Schmerzempfindungen auslösen.
Nach externer Stimulation werden ganze Entladungssalven statt einzelner Impulse an das Gehirn weitergeleitet (Herdegen und Zimmermann, 1995;
Herdegen-T; Zimmermann-M: Immediate eraly genes (IEGs) incoding inducible transcription factors (ITFs) and neuropeptides in the nervous system: functional networks for long-term plasticity and pain. In: Nyberg-F; Sharma-HS;
Wiesenfeld-Hallin-Z (Eds.): Neuropeptides in the spinal cord. Progress in Brain Research Vol 104 Elsevier Publishers, Amsterdam 1995, pp. 299-321).

Aufgrund der Beteiligung von Noradrenalinrezeptoren, dem Überträgerstoff des sympatischen Systems, spricht man auch von sympathisch unterhaltenem Schmerz, da durch physiologische Aktivierung des sympathischen Systems diese Neuronen aktiviert werden. Im englischen Sprachgebrauch ist daher der Begriff der "reflex sympathetic dystrophy" (RSD) (Rogers and Valley, 1994; Rogers-JN; Valley-MA, Reflex sympathetic dystrophy; Clin-Podiatr-Med-Surg. 1994 Jan; 11(1): 73-83) oder des "sympathetically maintained pain" (SMP) verbreitet.
Zytostatika wie Vincristin führen direkt zu einer Steigerung der Erregbarkeit von peripheren Schmerzrezeptoren und sollen auf diesem Wege die Hyperalgesie auslösen (Tanner et al. 1998; Tanner-KD; Reichling-DB; Levine-JD, Nociceptor hyper-responsiveness during vincristine-induced painful peripheral neuropathy in the rat. J-Neurosci. 1998 Aug 15; 18(16): 6480-91).

Tierexperimentell wurde versucht, grundlegende gemeinsame Mechanismen von Hyperalgesie aufzuklären. Wird in Ratten durch partielle Ligatur eines vom Rückenmark abgehenden Nerven eine peripher nachweisbare starke Hyperalgesie ausgelöst, dann sind im Rückenmark überaktive Neuronengruppen als ektopisch spontan aktive Herde aufzufinden (Pan et al., 1999; Pan-HL;
Eisenach-JC; Chen-SR, Gabapentin suppresses ectopic nerve discharges and reverses allodynia in neuropathic rats. J-Pharmacol-Exp-Ther. 1999 Mar; 288(3): 1026-30). Durch Gabapentin, ein Medikament mit ausgeprägter Wirkung bei neuropathischem Schmerz, läßt sich die spontane Aktivität dieser Nervenzellherde (ektopische foci) dosisabhängig unterdrücken. Im gleichen Dosisbereich wird auch die periphere Hyperalgesie unterdrückt. Ähnliche Versuche wurden auch in einem anderen Modell durchgeführt (Häbler et al., 1998; Häbler-HJ; Liu-XG; Eschenfelder-S; Jänig-W. Is sympathetic-sensory coupling in L5 spinal nerve-injured rats direct? Soc. Neurosci. Abstr. 24, 2084). Wurde der Spinalnerv L5 durchtrennt, dann konnte beginnend ab Tag 4 über mehrere Wochen hindurch unerwarteterweise spontane Aktivität einzelner Nervenfasern aus dem Nervenstumpf abgeleitet werden.

Dieses Phänomen ist möglicherweise dem Phantomschmerz zuzuordnen. Möglicherweise ist die Spontanaktivität dieser Nervenfasern nach Amputation auf eine Enthemmung des NMDA-Subtyps des Glutamatrezeptors zurückzuführen (Zhuo, 1998; Zhuo-M, NMDA receptor-dependent long term hyperalgesia after tail amputation in mice. Eur-J-Pharmacol. 1998 May 22; 349(2-3): 211-20.
Auf die Beteiligung des NMDA-Rezeptors deuten auch Untersuchungen, in denen gezeigt werden konnte, daß intrathekale Applikation von NMDA-Antagonisten den Schmerz zu reduzieren vermag. Es kann zusammenfassend festgestellt werden, daß Übererregungszustände der beteiligten Nerven als Ursache der Hyperalgesie bzw. der veränderten Schmerzwahmehmung eine Rolle spielen können, aber der Einfluß weiterer Faktoren wahrscheinlich ist.

Bei der Therapie dieser Erkrankungen muß ganz klar zwischen der symptomatischen Behandlung der Schmerzempfindung und der nervenzellschützenden Behandlung der Ursachen der Erkrankung unterschieden werden (Mörz 1999, Mörz-R; Schmerzbehandlung bei diabetischen Neuropathien Fortschritte der Medizin 1999, 13: 29-30).
Bei Patienten mit Diabetes-bedingtem neuropathischem Schmerz ist als Basisprogramm die Optimierung der Stoffwechsellage zur Vermeidung einer weiteren Progression und die Verhütung von Folgeschäden wie Fußläsionen angezeigt, diese Behandlung hat jedoch keinen Einfluß auf die Schmerzsymptome an sich.

Weiterhin kann die Ursache der Erkrankung, d.h. die neurodegenerative Nervenschädigung und die zugrundeliegende Mikroangiopathie durch Einsatz von nervenzellschützenden (neuroprotektiven) Substanzen wie Alpha-Liponsäure oder andere Antioxidantien wie Vitamin E, für das Nervensystem relevante Vitamine wie Vit. B1, B6 oder B12 oder durch die Durchblutung verbessernde Maßnahmen wie körperliches Training behandelt werden.

Diese Art der Behandlung beeinflußt akut die Schmerzen nicht, wenn jedoch eine Verbesserung der Nervenfunktion erreicht wird, ist es möglich, daß die Schmerzempfindungen langfristig nachlassen.

Die eigentliche symptomatische Schmerztherapie dagegen muß auf andere Medikamente zurückgreifen. Weder zentral wirksame Analgetika wie Morphin-Derivate noch übliche peripher wirksame Analgetika wie Paracetamol oder Acetylsalicylsäure sind wirksam. Es werden dagegen Antidepressiva wie Amitriptylin, Imipramin oder Paroxetin oder Antikonvulsiva wie Carbamazepin oder Gabapentin eingesetzt. Tramadol als Opioid-Analgetikum ist aufgrund seiner weiteren Wirkungen auf andere Rezeptoren des adrenergen Systems auch wirksam.

In der Patentliteratur wird beispielsweise die Verwendung von Topiramat (US 5 760 007) und Moxonidin (EP 901 790) zur Behandlung von neurophatischen Schmerzen aufgezeigt.

Ziel ist dabei, die Schmerzsymptome an sich und nicht die Ursachen zu behandeln. Alle angeführten Medikamente führen aber nur bei einem Teil der Patienten zu einer Linderung der Schmerzsymptome.
Bei Herpes-induziertem neuropathischem Schmerz kann prophylaktisch durch den Einsatz von Virostatika zu einem frühen Zeitpunkt der Erkrankung die Nervenzelle ursächlich vor der schädigenden Wirkung des Virus geschützt und dadurch die Ausprägung der neuropathischen Schmerzen reduziert werden, diese Medikamente sind jedoch nicht symptomatisch nach Abklingen der akuten Infektion wirksam. Betroffene Patienten können durch Einnahme von Antidepressiva, Carbamazepin oder Gabapentin eine Linderung der Symptome erfahren.

Bei kompressionsbedingten neuropathischen Schmerzen kann z.B. beim Carpaltunnel-Syndrom oder bei Kompression von Rückenmarkswurzeln durch chirurgische Erweiterung der Engstellen die primäre Ursache der Erkrankung beseitigt werden. Neuroprotektiv wirkende Medikamente können bei frühzeitiger Gabe die Progression der Schädigung der Nerven verzögern oder aufhalten. Trotzdem leidet ein hoher Anteil dieser Patienten auch lange Zeit nach der Operation noch an Schmerzen, die wiederum nicht gut auf klassische Analgetika ansprechen. Es kommen Antidepressiva und Medikamente wie Carbamazepin oder Gabapentin zum Einsatz.
Bei Amputationsschmerz ist die eigentliche Ursache, die Amputation, nicht zu behandeln, so daß neuropathische Schmerzen nur symptomatisch mit den oben erwähnten Medikamentengruppen behandelt werden müssen. Jedoch wird in der letzten Zeit versucht, bei planmäßigen Amputationen durch mehrtägige Leitungsblockade der zu durchtrennenden Nerven vor Durchführung der Amputation der Entwicklung von neuropathischen Schmerzen entgegenzuwirken. Obwohl erste Hinweise positiv sind, ist der eindeutige Wirksamkeitsnachweis in kontrollierten klinischen Studien noch nicht erbracht.

Zusammenfassend kann festgestellt werden, daß zur symptomatischen Behandlung von neuropathischen Schmerzen klassische Analgetika eine geringe Wirksamkeit aufweisen. Es kommen Medikamente wie Antidepressiva, Carbamazepin oder Valproat zum Einsatz, die an sich keine analgetische Wirkung auf nicht neuropathische Schmerzformen aufweisen.
Die Behandlung dieser Patienten ist jedoch oft nicht befriedigend.

Es besteht daher ein großer Bedarf nach neuen Substanzen zur selektiven Behandlung von bestimmten neuropathischen Schmerzformen.

Überraschenderweise wurde nun gefunden, daß Retigabin der Formel 1 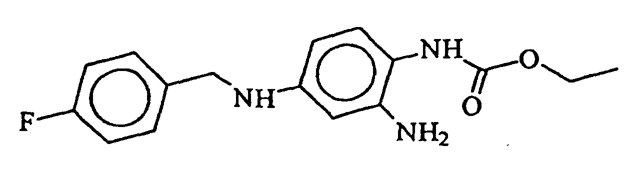 bedeutende Wirksamkeiten gegen bestimmte neuropathische Schmerzen, wie in den Ansprüchen angegeben, aufweist. Damit öffnen sich völlig neue Möglichkeiten für die Prophylaxe und Behandlung der angegebenen neuropathischen Schmerzen.

Retigabin und Verfahren zu seiner Darstellung sind bekannt (DE 42 00 259).

Retigabin ist ein Derivat des nicht opioiden Analgetikums Flupirtin, für das bereits neben seiner analgetischen Wirkung auch eine antikonvulsive Wirkung nachgewiesen werden konnte. Durch Strukturoptimierung mit dem Ziel der besseren Trennung von erwünschten (antikonvulsiven) Eigenschaften von unerwünschten (analgetischen) Wirkungen konnte unter Verwendung von Pharmakophor-Modellierungen die antikonvulsive von der analgetischen Wirkung in dieser Substanzklasse getrennt werden.
Retigabin wirkt stärker antikonvulsiv als Flupirtin, eine analgetische Wirkung in Modellen des akuten Schmerzes ist dagegen nicht mehr nachweisbar (Rostock et al., 1996; Rostock-A; Tober-C; Rundfeldt-C; Bartsch-R; Engel-J; Polymeropoulos-EE; Kutscher-B; Löscher-W; Honack-D; White-HS; Wolf-HH D-23129: a new anticonvulsant with a broad spectrum activity in animal models of epileptic seizures. Epilepsy-Res. 1996 Apr; 23(3): 211-23).

Retigabin weist ein breites Wirkungsspektrum in experimentellen Modellen epileptischer Anfälle auf (Rostock et al., 1996; Tober et al., 1996; Tober-C; Rostock-A; Rundfeldt-C; Bartsch-R D-23129: a potent anticonvulsant in the amygdala kindling model of complex partial seizures. Eur-J-Pharmacol. 1996 May 15; 303(3): 163-9) und befindet sich in der klinischen Entwicklung für die Behandlung der Epilepsie.

Desweiteren ist die Verwendung von Retigabin zur Behandlung von neurodegenerativen Erkrankungen in EP 857 065 beschrieben.

Unerwarteterweise konnten wir feststellen, daß Retigabin eine deutliche dosisabhängige Wirkung gegen neuropathische Schmerzen aufweist.
Wie erwartet, war dagegen die analgetische Wirkung, wie sich in diesem Modell in der Frühphase zeigt, nur gering und vergleichbar mit der Referenzsubstanz Gabapentin.

### Pharmakologische Untersuchungen

### Untersuchung der Hemmung der Hyperalgesie am Formalin-Modell der Ratte

In diesem Modell wird durch die subkutane Injektion von niederprozentigem Formalin plantar in der Hinterpfote eine biphasische nocifensive Verhaltensreaktion ausgelöst (Field et al. 1997; Field-MJ; Oles-RJ; Lewis-AS; McCleary-S; Hughes-J; Singh-L, Gabapentin (neurontin) and S-(+)-3-isobutylgaba represent a novel class of selective antihyperalgesic agents. Br-J-Pharmacol. 1997 Aug; 121(8): 1513-22). Die frühe Phase bis spätestens zur 10. Minute ist durch intensives Lecken und Beißen charakterisiert. Die späte tonische Phase tritt 20 - 60 min. nach der Injektion auf als Stadium des hyperalgetischen Prozesses. Die Formalin-induzierte Hyperalgesie schließt zentrale Mechanismen über eine Sensitivierung der Neuronen im Dorsalhorn des Rückenmarks ein, die infolge der Gewebsschädigung oder zunehmender Aktivierung der C-afferenten Fasern entsteht.

Field et al (1997) konnten nachweisen, daß Opioide an diesem Modell gegen die späte Phase der hyperalgetischen Verhaltensreaktionen unwirksam sind.

Hingegen reduzierte das Antikonvulsivum Gabapentin dosisabhängig die Schmerzreaktionen der Ratten.

### Untersuchungen mit Retigabin

### Methode:

Männliche Sprague Dawley Ratten im Gewicht von 70 - 90 g wurden einzeln adaptiert und mindestens 15 min vor Versuchsbeginn beobachtet. 0,05 ml 2,5 % Formaldehyd in isotonischer Kochsalzlösung plantar subkutan in die Hinterpfote injiziert bewirkte eine starke Sofortreaktion mit Beißen und Lecken von wenigen Minuten Dauer mit nachfolgend hyperalgetischer Spätphase bis zu 60 min nach Formalin-Injektion. Die Beiß- und Leckreaktionen der Spätphase (beginnend ab 10 min) bilden das Maß der hyperalgetischen Reaktionen. Diese Reaktionen werden in 5 Minuten-Intervallen über 40 min erfaßt. Die Testsubstanzen wurden oral 60 min vor der Formalin-Injektion verabreicht. Referenzsubstanz war Gabapentin. Pro Gruppe wurden 15 Tiere eingesetzt.

### Ergebnis:

Retigabin hemmte die als Hyperalgesie oder neuropathischer Schmerz zu bezeichnende Spätphase der Schmerzreaktionen dosisabhängig nach 5, 10 und 20 mg/kg oral. Die Wirkung von 10 mg/kg Retigabin entsprach etwa dem Effekt von 60 mg/kg oral Gabapentin (siehe Tabelle 1).

Wie bei Gabapentin war die Wirkung auf die Frühphase des Schmerzes, d.h. die analgetische Wirkung, nur gering. So erreichten die aufsummierten Schmerzreaktionen in der Kontrollgruppe in der Frühphase einen Verhaltensscore von bis zu 35 und in der Spätphase von bis zu 30. Durch Retigabin wurde die Schmerzreaktion in der Frühphase dosisabhängig auf 32, 25 und 18 reduziert, wohingegen in der Spätphase mit Verhaltensscores teilweise unter 5 fast keine Schmerzreaktion mehr nachweisbar war.
Auch durch Gabapentin wurde die Schmerzreaktion der Frühphase nur auf 27 gesenkt, wohingegen in der Spätphase auch Werte von unter 6 erreicht wurden.

**Tabelle 1**

| Effekt von Retigabin auf die Hyperalgesie von Ratten nach oraler Gabe | | | | | | | | | | |
|---|---|---|---|---|---|---|---|---|---|---|
| | **Behandlung** **Zeit** | **Dosis m** **g/kg** | **Summe der Verhaltens-Scores über 5 min gemittelt beginnend ab Fomrmalin-Applikation (Mittelwert ± Standardabweichung)** | | | | | | | |
| | | | 5 | 10 | 15 | 20 | 25 | 30 | 35 | 40 |
| 1 | Vehicle | - | 35,3 ±2,76 | 14,9 ±7,00 | 24,0 ±7,78 | 30,1 ±3,91 | 28,9 ±7,08 | 23,9 ±7,90 | 23,8 ±7,61 | 17,5 ±11,40 |
| 2 | Retigabin | 5,0 | 31,9 ±5,46 | 5,33** ±6,43 | 9,9** ±8,51 | 26,4 ±6,63 | 21,1 ±6,15 | 20,6 ±7,85 | 14,5* ±7,80 | 10,9 ±8,98 |
| 3 | Retigabin | 10,0 | 25,1** ±6,42 | 2,5** ±2,78 | 5,6** ±3,46 | 12,9** ±8,49 | 16,5** ±10,49 | 8,8** ±8,91 | 8,4** ±8,47 | 8,3* ±7,29 |
| 4 | Retigabin | 20,0 | 17,6** ±8,52 | 0,9** ±1,81 | 5,4** ±8,47 | 12,5** ±8,83 | 9,3** ±9,18 | 9,0** ±8,70 | 7,9** ±5,91 | 2,9** ±3,36 |
| 5 | Gabapentin | 60,0 | 26,5⁺⁺ ±6,12 | 4,6⁺⁺ ±4,53 | 4,5⁺⁺ ±5,76 | 12,4⁺⁺ ±7,11 | 11,5⁺⁺ ±7,27 | 8,4⁺⁺ ±7,76 | 6,8⁺⁺ ±8,76 | 5,6⁺ ±9,21 |
| Statistische Unterschiede im Vergleich zur Vehicle-behandelten Gruppe wurden für Retigabin mittels Varianzanalyse mit nachfolgendem Williams' Test durchgeführt (*p<0,05, **p<0,01) | | | | | | | | | | |
| Statistische Unterschiede im Vergleich zur Vehicle-behandelten Gruppe wurden für Retigabin mittels Student's t Test durchgeführt (⁺p<0,05, ⁺⁺p<0,01). | | | | | | | | | | |

Retigabin der Formel I kann in bekannter Weise in pharmazeutische Formulierungen wie Tabletten, Kapseln, Dragees, Pillen, Granulate, Sirupe, Emulsionen, Suspensionen und Lösungen gegebenenfalls unter Verwendung pharmazeutisch geeigneter Träger- und/oder Hilfsstoffe überführt werden.

Bei oraler oder parenteraler Gabe sollte die Tagesdosis der Verbindung der Formel I 50 - 500 mg betragen.
Vorzugsweise werden Einzeldosen von 30 - 60 mg bei oraler Applikation und 5 - 20 mg bei parenteraler Applikation verabreicht (Die Mengen sind jeweils bezogen auf die freie Base).
Erforderlichenfalls kann von den genannten Mengen abgewichen werden und zwar in Abhängigkeit vom Körpergewicht und der speziellen Art des Applikationsweges.

## Patentansprüche

1. Verwendung der Verbindung I 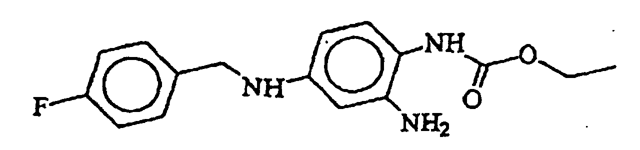 oder deren pharmazeutisch verwendbaren Salze zur Herstellung eines Arzneimittels zur Prophylaxe oder Behandlung von Allodynie, wobei es sich bei der Allodynie nicht um postherpetische Neuralgie handelt.

2. Verwendung der in Anspruch 1 genannten Verbindung der Formel I zur Prophylaxe oder Behandlung von hyperalgetischen Schmerzen, wobei es sich bei den hyperalgetischen Schmerzen nicht um Trigeminusneuralgie oder Schmerzen nach Verbrennungen handelt.

3. Verwendung der in Anspruch 1 genannten Verbindung der Formel I zur Prophylaxe oder Behandlung von Phantomschmerzen.

4. Verwendung der in Anspruch 1 genannten Verbindung der Formel I zur Prophylaxe oder Behandlung von neuropathischem Schmerz bei diabetischer Neuropathie.

5. Verwendung der in Anspruch 1 genannten Verbindung der Formel I zur Prophylaxe oder Behandlung von Schmerzen des Krankheitsbildes der RSD (Reflex Sympathetic Dystrophy).

6. Verwendung der in Anspruch 1 genannten Verbindung der Formel I zur Prophylaxe oder Behandlung von Vulvodynie.

7. Verwendung der in Anspruch 1 genannten Verbindung der Formel I zur Prophylaxe oder Behandlung von Parästhesien.

8. Verwendung der in Anspruch 1 genannten Verbindung der Formel I zur Prophylaxe oder Behandlung von kompressionsbedingten neuropathischen Schmerzen.

9. Verwendung der in Anspruch 1 genannten Verbindung der Formel I zur Prophylaxe oder Behandlung von neuropathlschen Schmerzen in Folge von Rückenmarksverletzungen.

## Claims

1. Use of compound I 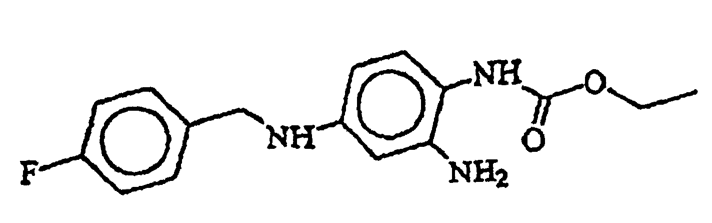 or of its pharmaceutically usable salts for the preparation of a pharmaceutical composition for the prophylaxis or treatment of allodynia, wherein allodynia is not post-herpetic neuralgia.

2. The use of the compound of formula I as indicated in claim 1 for the prophylaxis or treatment of hyperalgetic pain, wherein the hyperalgetic pain is not trigeminal neuralgia or pain after a burn.

3. The use of the compound of the formula I as indicated in claim 1 for the prophylaxis or the treatment of pseudoesthesia.

4. The use of the compound of the formula I as indicated in claim 1 for the prophylaxis or the treatment of neuropathic pain in the case of diabetic neuropathy.

5. The use of the compound of the formula I as indicated in claim 1 for the prophylaxis or the treatment of pain of the clinical picture of RSD (reflex sympathetic dystrophy).

6. The use of the compound of the formula I as indicated in claim 1 for the prophylaxis or the treatment of vulvodynia.

7. The use of the compound of the formula I as indicated in claim 1 for the prophylaxis or the treatment of paresthesiae.

8. The use of the compound of the formula I as indicated in claim 1 for the prophylaxis or the treatment of neuropathic pain caused by compression.

9. The use of the compound of the formula I as indicated in claim 1 for the prophylaxis or the treatment of neuropathic pain caused by spinal injury.

## Revendications

1. Utilisation du composé I 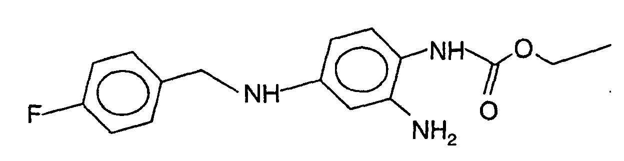 ou de ses sels pharmaceutiquement utilisables pour la préparation d'un médicament pour la prophylaxie ou le traitement de l'allodynie, l'allodynie n'étant pas une névralgie post-herpétique.

2. Utilisation du composé de formule I selon la revendication 1 pour la prophylaxie ou le traitement des douleurs hyperalgésiques, les douleurs hyperalgésiques n'étant pas une névralgie essentielle du trijumeau ou des douleurs consécutives à des brûlures.

3. Utilisation du composé de formule I selon la revendication 1 pour la prophylaxie ou le traitement d'illusions des amputés.

4. Utilisation du composé de formule I selon la revendication 1 pour la prophylaxie ou le traitement de la douleur névropathique dans la neuropathie diabétique.

5. Utilisation du composé de formule I selon la revendication 1 pour la prophylaxie ou le traitement de douleurs du tableau morbide de la RSD (Reflex Sympathetic Dystrophy).

6. Utilisation du composé de formule I selon la revendication 1 pour la prophylaxie ou le traitement de la vulvodynie.

7. Utilisation du composé de formule I selon la revendication 1 pour la prophylaxie ou le traitement des paresthésies.

8. Utilisation du composé de formule I selon la revendication 1 pour la prophylaxie ou le traitement des douleurs névropathiques dues à des compressions.

9. Utilisation du composé de formule I selon la revendication 1 pour la prophylaxie ou le traitement des douleurs névropathiques consécutives à des lésions de la moelle épinière.
